# NEW EUROPEAN PATENT SPECIFICATION

(11) **EP 1 673 060 B2**
(45) Date of publication and mention of the opposition decision: **25.09.2013**
(45) Mention of the grant of the patent: 16.12.2009
(21) Application number: 04817261.3
(22) Date of filing: 23.09.2004
(51) Int. Cl.: A61K 8/18, A61K 8/25, A61Q 15/00

(54) **ANTIPERSPIRANT SPRAY COMPOSITIONS**
ANTIPERSPIRANS-SPRAY-ZUSAMMENSETZUNGEN
COMPOSITIONS ANTISUDORIFIQUES AEROSOLS

(30) Priority: 13.10.2003 GB 0323958
(43) Date of publication of application: 28.06.2006
(73) Proprietor: Unilever PLC, London EC4Y 0DY (GB); Unilever N.V., 3013 AL Rotterdam (NL)
(72) Inventor: FRANKLIN, Kevin, Ronald, Bebington, Wirral Merseyside CH63 3JW (GB); GREEN, Helena, Wollaton, Nottinghamshire (GB)
(74) Representative: Whaley, Christopher
(86) International application number: PCT/EP2004/010738
(87) International publication number: WO 2005/039519

(56) References cited:
- EP-A- 0 279 328
- WO-A-99/17718
- WO-A-03/000218
- WO-A-03/105795
- WO-A1-96/24326
- WO-A1-03/041674
- GB-A- 2 113 116
- GB-A- 2 113 116
- US-A- 4 680 173

## Description

This invention relates to cosmetic antiperspirant spray compositions suitable for topical application to the human body and to methods of making the same.

Antiperspirant spray compositions suitable for topical application typically comprise an antiperspirant active such as aluminium chlorohydrate, which acts to suppress the level of perspiration on the area of the body to which it is applied. Unfortunately, topical application of such actives, and/or application of materials with which they are formulated, can be perceived as unpleasant by some individuals. Overcoming such sensory negatives and delivering a good antiperspirancy benefit are both objectives of the present invention.

The present invention relates to cosmetic antiperspirant spray compositions in which the antiperspirant active material is present as droplets of an aqueous solution emulsified in an oily continuous phase, the composition further comprising a particular sensory modifier. Spray compositions of this general type, i.e. lacking the particular sensory modifier of the present invention, are known in the prior art.

GB 2,113,116 A discloses suspensions of oily liquid in polar liquid stabilised by means of a hydrophobic oxide.

US 4,695,451 (Straw et al) discloses an antiperspirant aerosol composition in the form of a substantially stable water-in-oil emulsion. The compositions of the disclosed invention comprise an emollient oil which is claimed to provide a desirable film having an emollient or lubricating effect on the skin.

EP 812,182 B1 (Correia) discloses an antiperspirant aerosol composition comprising a base in the form of a water-in-oil emulsion stabilised by a silicone surfactant and packaged in an aluminium can. In a highly preferred aspect of the disclosed invention, the base comprises an emollient, preferably a C₂-C₄ polyol emollient. Skin feel improvers such as talc and finely divided polyethylene may also be employed.

The article "New Approaches to Antiperspirant and Deodorant Formulation" by A. J. Disapio in HAPPI, February 1986, discloses the use of silicone oils in water-in-oil antiperspirant aerosol emulsion compositions.

WO 94/22420 (Shaw) discloses silicone-based water-in-oil micro-emulsions that employ a silicone emulsifying agent and various polyols that could theoretically act as emollients.

EP 373,424 B1 (Raleigh et al) discloses an antiperspirant composition in the form of a water-in-oil emulsion stabilised by a silicone surfactant and an organic surfactant.

US 4,264,586 (Callingham et al) discloses an antiperspirant composition suitable for spray application that is in the form of a water-in-oil emulsion comprising a wax dissolved in polydimethylsiloxane. The optional presence of an additional emollient oil is also disclosed.

US 4,268,499 (Keil) disclose an antiperspirant composition in the form of a water-in-oil emulsion having methylsiloxane fluid as the continuous phase and stabilised by a three-component emulsifying system.

WO 99/17718 (Brewster) discloses an antiperspirant aerosol composition including a propellant, a C1-C3 alcohol and an antiperspirant aluminium astringent salt having a water loss under drying no greater than about 8%. Clays and colloidal pyrogenic silica are disclosed as preferred materials for use as bulking/suspending agents.

According to a first aspect of the present invention, there is provided an antiperspirant spray composition comprising an aqueous solution of antiperspirant active emulsified in an oily continuous phase with an emulsifier that is a nonionic surfactant, characterised in comprising a hydrophobically-modified particulate silica.

According to a second aspect of the present invention, there is provided a cosmetic method of delivering an antiperspirancy benefit comprising the spray application to the human body of a composition according to the first aspect of the present invention or any of the particular variants thereof described herein.

According to a third aspect of the present invention, there is provided a method of manufacture of an antiperspirant spray composition comprising the emulsification of an aqueous solution of antiperspirant active in an oily continuous phase using an emulsifier that is a nonionic surfactant, a hydrophobically-modified particulate silica being added during the manufacture.

Antiperspirant spray compositions should be understood to refer to Antiperspirant compositions that are suitable for spray application. Such compositions may be converted into spray form by any of the methods known in the art (*vide infra*).

The antiperspirancy benefit delivered in accordance with the present invention is generally accompanied by a sensory benefit resulting from the presence of the hydrophobically-modified particulate silica. This sensory benefit may be in the form of a reduced sensory negative or, preferably, it may be in the form of a positive sensory benefit, i.e. a sensory attribute perceived as positively desirable by the user.

Emulsion compositions comprising water suffer from the disadvantage that they can lead to a cool feel on application to the skin and/or a wet feel on application to the skin and/or shortly thereafter. The emulsion compositions of the present invention may deliver the particular sensory benefits of reduced coolness on application and/or reduced wetness on application and/or shortly thereafter. In addition, the visual appearance of compositions of the present invention, when applied to the skin surface, may be better than analogous emulsion compositions of the prior art; in particular, the compositions of the present invention may appear less wet and shiny than those of the prior art.

A further advantage of the present invention is that the emulsion compositions involved have good phase stability and do not readily separate on storage. In addition, when there is some phase separation, the aqueous phase may be easily re-dispersed, by gentle shaking of the composition for example. It is believed that the hydrophobically-modified particulate silica together with the emulsifier may contribute to this phase stability and/or re-dispersibility.

A further advantage of the present invention is that the emulsion compositions deliver good antiperspirancy. It is hypothesised that the hydrophobically-modified particulate silica may assist by aiding delivery of the antiperspirant (AP) active from the internal phase of the emulsion onto the skin of the user. A different hypothesis for the superior antiperspirancy of the compositions of the present invention is that the hydrophobically-modified particulate silica may co-operate with the AP active in the formation of a 'plug' in the opening of the sweat glands on the surface of the body. These two hypotheses are not, of course, mutually exclusive and either or both may be involved in delivering the observed antiperspirancy benefit.

AP actives for use herein are typically selected from astringent salts including, in particular, aluminium, zirconium, and mixed aluminium-zirconium salts, including both inorganic salts, salts with organic anions, and complexes. Preferred AP salts are aluminium, zirconium, and aluminium-zirconium chlorides, oxychlorides, and chlorohydrates salts. Particularly preferred AP salts are polynuclear in nature, meaning that the cations of the salt are associated into groups comprising more than one metal ion.

Aluminium halohydrates are preferred AP actives and may be defined by the general formula Al₂(OH)ₓQ_{y}.wH₂0, in which Q represents chlorine, bromine or iodine, x is variable from 2 to 5 and x + y = 6 while wH₂O represents a variable amount of hydration. Aluminium chlorohydrate (ACH) is an especially preferred active.

Zirconium salts are usually defined by the general formula ZrO(OH)₂₋ₓQₓ.wH₂O in which Q represents chlorine, bromine or iodine; x is from about 1 to 2; w is from about 1 to 7; and x and w may both have non-integer values. Preferred are zirconyl oxyhalides, zirconiun hydroxyhalides, and combinations thereof. Non-limiting examples of zirconium salts and processes for making them are described in Belgian Patent 825,146, Schmitz, issued August 4, 1975 and U.S. Patent 4,223,010 (Rubino).

AP actives as used in the invention may be present as mixtures or complexes. Suitable aluminium-zirconium complexes often comprise a compound with a carboxylate group, for example an amino acid. Examples of suitable amino acids include tryptophan, β-phenylalanine, valine, methionine, β-alanine and, most preferably, glycine.

In some embodiments, it is desirable to employ complexes of a combination of aluminium halohydrates and zirconium chlorohydrates with amino acids such as glycine, which are disclosed in US 3,792,068 (Procter and Gamble Co.). Certain of these Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine.

Other actives that may be utilised include astringent titanium salts, for example those described in GB 2,299,506.

AP actives are preferably incorporated into compositions of the invention in an amount of from 0.5 to 60%, particularly from 5 to 30% or 40% and especially from 5 or 10% to 30 or 35% by weight.

The AP active is generally dissolved in water prior to emulsification. The aqueous AP active solution that is emulsified is preferably of concentration from 10% to 70%, more preferably from 25% to 60%, and most preferably from 30% to 50% by weight, these preferred concentrations aiding product efficacy.

For stability and efficacy reasons, the water-in-oil emulsion AP compositions of the present invention preferably have a proportion of dispersed phase of from 50% to 90%, more preferably from 60% to 80%, and most preferably from 65% to 75% by weight of the composition excluding any volatile propellant that may be present. The presence of the hydrophobically-modified particulate silica is particularly valuable in compositions having these higher levels of dispersed phase.

The droplets of aqueous phase dispersed in the oily continuous phase typically have a relative small particle size, at least 90% of the droplets being from 1 to 25 µm, in particular from 1 to 10 µm, and especially from 1 to 7 µm in diameter. Particle size determination may be performed using optical microscopy and appropriate image analysis techniques.

The oily continuous phase typically comprises a silicone oil, a hydrocarbon oil, an ester oil, or any mixture thereof. When more than one oil is present, it is preferred that the oils are miscible in order to avoid the need to shake the composition before application. It is preferred that compositions of the invention comprise a silicone oil in the oily continuous phase. Silicone oils may be cyclic or linear, examples include Dow Corning silicone fluids 344, 345, 244, 245, 246, 556, 704, and the 200 series; Union Carbide Corporation Silicones 7207 and 7158; and General Electric silicone SF1202. Alternatively or additionally, non-silicone oils may be used; such materials include mineral oils, hydrogenated polyisobutene, polydecene, paraffins, isoparaffins of at least 10 carbon atoms, liquid fatty alcohols (e.g. isosteayl alcohol or octyl dodecanol), and aliphatic or aromatic ester oils (e.g. isopropyl myristate, lauryl myristate, isopropyl palmitate, diisopropyl sebecate, diisopropyl adipate, or C₈ to C₁₈ alkyl benzoates.

The oily continuous phase preferably comprises from 10% to 50%, more preferably from 20% to 40%, and most preferably from 25% to 35% by weight of the composition excluding any volatile propellant that may be present.

The hydrophobically-modified particulate silica is an essential component of the present invention. It is at its most effective when at least part of it is suspended in the oily continuous phase. Suitable silicas are surface-modified to increase their hydrophobicity. Silylation is a technique that may be used to increase the hydrophobicity of the silica, the resulting silicas having terminal polydimethylsiloxane groups and/or trimethylsilane groups. Partially hydrophobed silicas are preferred, in particular partially-silylated silicas, and especially partially-silylated silicas having a degree of silylation of from 25% to 75%. The degree of silylation represents the number of silanol (Si-OH) groups of the silica that have been modified by the hydrophobing treatment. Preferred hydrophobically-modified silicas are based on fumed silica. Preferred hydrophobically-modified are organo-silylated. The carbon content of the hydrophobically-modified silica is preferably from 0.5% to 2.5% by weight. The preferred silicas give particularly good wetness reduction benefits.

For optimum sensory benefit, the hydrophobically-modified particulate silica is typically used at from 0.1% to 3%, in particular at from 0.15% to 1%, and especially at from 0.2% to 0.7% by weight of the total composition. At low levels of hydrophobically-modified particulate silica, the sensory benefit is less noticeable, whilst at high levels there can be a problem with white deposits on the skin and/or clothing that comes into contact with treated skin. In addition, compositions comprising the preferred levels of hydrophobically-modified particulate silica can be more readily processed than compositions comprising higher levels. The most preferred level of hydrophobically-modified particulate silica is from 0.3% to 0.4% by weight of the total composition.

An emulsifier that is a nonionic surfactant is required to stabilise the emulsion compositions of the present invention. The emulsifier has an amphiphilic molecular structure. The proportion of emulsifier may be from 0.05% to 5%, preferably from 0.1% to 2.5%, and more preferably from 0.15% to 0.5% by weight of the composition. The preferred levels indicated are the best for phase stability of the emulsion products - they reduce droplet sedimentation even in the presence of the hydrophobically-modified silica, which can otherwise make this problem particularly severe.

It is desirable to use an emulsifier or a mixture of emulsifiers with an overall HLB value in a range from 2 to 10, preferably from 3 to 8. A mixture of emulsifiers may comprise a surfactant of high HLB and a surfactant of low HLB, blended to give a suitable overall HLB.

High HLB emulsifiers include nonionic esters or ethers comprising a polyoxyalkylene moiety, especially a polyoxyethylene (POE) moiety containing from 2 to 80, and especially from 5 to 60, ethylene oxide (EO) units. Polyoxypropylene (POP) emulsifiers may also be employed, as may emulsifiers comprising one or more polyhydroxylated units such as glycerol, sorbitol, or some other alditol. The emulsifier must also comprise a hydrophobic moiety, for example an alkyl, alkenyl, or aralkyl group, normally containing from about 8 to 50 carbons and particularly from 10 to 30 carbons. The hydrophobic moiety can be either linear or branched and is often saturated, though it can be unsaturated, and it is optionally fluorinated. The hydrophobic moiety can comprise a mixture of chain lengths, for example those deriving from tallow, lard, palm oil, sunflower seed oil or soya bean oil. Examples of suitable high HLB emulsifiers include C₁₆ to C₁₈ alcohols ethoxylated with 10 to 25 ethylene oxide residues and PEG-15-25 stearate or distearate. Other suitable examples include C₁₀-C₂₀ fatty acid mono, di or tri-glycerides. Further examples include C₁₈-C₂₂ fatty alcohol ethers of polyethylene oxides with 8 to 12 EO units.

Low HLB emulsifiers, typically of HLB from 2 to 6, include fatty acid mono- or possibly di-esters of polyhydric alcohols such as glycerol, sorbitol, erythritol or trimethylolpropane. The fatty acyl moiety is often from C₁₄ to C₂₂ and is saturated in many instances, including cetyl, stearyl, arachidyl and behenyl. Examples include monoglycerides of palmitic or stearic acid, sorbitol mono or diesters of myristic, palmitic or stearic acid, and trimethylolpropane monoesters of stearic acid.

Emulsifiers that are silicone derivatives, by which it is meant emulsifiers that have a lipophilic silicone chain, are particularly preferred, especially when the oily continuous phase of the composition comprises a silicone oil. Examples of such emulsifiers include polyoxyalkylene derivatives of dimethylpolysiloxanes, in particular POE, POP, or POE-co-POP derivatives. Such derivatives may terminate in C₁ to C₁₂ alkyl groups. Such emulsifiers may also be dimethicone copolyol silicone surfactants, for example cetyl dimethicone copolyol (sold as Abil EM90, ex Goldschmidt) or lauryl dimethicone copolyol (sold as DC 5200, *ex* Dow Corning). Emulsifiers that are silicone derivatives are preferably used at from 0.1 to 5%, more preferably 0.15 to 2.5%, and most preferably at from 0.25 to 0.4% by weight of the composition. The preferred levels indicated are the best for phase stability of the emulsion products.

The oily continuous phase may comprise an emollient oil as one of its component oils. Suitable emollient oils are disclosed in US Patent Nos. 4,822,596 and 4,904,463. Preferred emollient oils are alkyl esters, such as PurSyn Ester 2E7 (neopentylglycol dihexanoate), ex Exxon-Mobil; benzoate esters such as Finsolv TN (Trade Mark), ex Finetex Inc.; hydrogenated polyalkenes, such as Panalane, ex Amoco and PureSyn PAO 2 (hydrogenated polydecene), ex Exxon-Mobil; PPG ethers, such as Fluid AP (PPG-14 butylether), ex Union Carbide; isopropyl palmitate; phenylsilicone; and isopropyl myristate.

In order to further enhance phase stability and/or re-dispersibility, an additional suspending agent may be incorporated into the compositions of the invention. The additional suspending agent may be a hydrophobically treated montmorillionite clay such as a bentonite or a hectorite. One such commercially available clay is Bentone 38V, which is a hectorite clay available from NL Industries, Inc. The amount of clay in the composition of the invention may be from 0.2 to 5.0% by weight of the total composition.

In certain preferred embodiments of the invention a polymeric co-gellant for the antiperspirant active is employed, as described in WO 02/49590 (Smith et al) and WO 03/105795 (Brown et al). Such polymeric co-gellants comprise Bronsted acid groups and act as co-gellants for the AP salt when mixed therewith in the presence of water, for example water in human sweat, at a temperature of 37°C or less. The polymeric co-gellant may be present as a particulate solid suspended in the oily continuous phase or, preferably, as an aqueous solution emulsified as a separate dispersed phase. When present as an aqueous solution emulsified as a separate disperse phase, it is preferably used as a solution of concentration from 5% to 50%, more preferably from 10% to 30%, and most preferably from 15% to 20% by weight.

When used, the polymeric co-gellant is preferably incorporated into the composition in an amount of from 0.05% to 10%, more preferably from 0.2% to 5% by weight, and most preferably from 1% to 4% by weight of said composition, excluding any volatile propellant present.

The compositions of the invention may be turned into spray form by any of mechanical means known in the art. In particular they may be used as squeeze-spray products or trigger-spray products, in combination with a suitable dispenser. Alternatively, the compositions according to the invention may use compressed gas or may comprise a volatile propellant and be aerosol compositions.

The compositions of the invention may be packaged in plastic containers, in order to avoid corrosion problems. For aerosol compositions, metal cans, for example tin-plate or, more preferably, aluminium may be used. Lacquered cans are particular preferred for packaging aerosol compositions.

Suitable volatile propellants for use in aerosol compositions according to the invention include trichlorofluoromethane, trichlorotrifluoromethane, difluoroethane, propane, butane or isobutane or combinations thereof. When used, the amount of liquefied gas in the composition of the invention may be from 5 to 95% and preferably from 30 to 90% by weight of the composition. When low VOC content is desired, the volatile propellant is preferably from 30 to 60%, more preferably from 30 to 50% by weight of the total composition.

Other minor ingredients which may be present in the compositions of the invention include:
- cosmetically acceptable carrier fluid components, such as straight and branched chain alcohols, for example, ethanol, isobutanol or isopropanol;
- deodorant active perfumes and deodorant compounds which can act as antimicrobial agents;
- inorganic electrolytes, such as sodium chloride or sodium sulphate;
- other rheology modifiers, such as hydroxypropyl celluloses;
- a silicone gum, such as Q2 1501, ex Dow Corning;
- polar additives such as propylene carbonate;
- additional skin feel improvers, such as talc and finely divided polyethylene such as Accumist B18;
- humectants, such as polyols, for example glycerol;
- perfumes;
- preservatives and antioxidants;
- skin benefit agents such as allantoin;
- colourants;
- other cosmetic adjuncts conventionally employed in
- propellant driven aerosol products.

The compositions of the invention may be manufactured by a method comprising the emulsification of an aqueous solution of antiperspirant active in an oily continuous phase using an emulsifier, a hydrophobically-modified particulate silica being added during the manufacture. Typically the aqueous solution of antiperspirant active is added to the oily continuous phase with stirring. Addition of the hydrophobically-modified silica is typically to the oily continuous phase of the emulsion once formed, preferably this addition is accompanied by vigorous mixing, as this addition often causes an increase in the viscosity of the product.

When a polymeric co-gellant is employed, the composition is preferably prepared as two separate emulsions, one comprising a water-in-oil emulsion of an aqueous solution of the AP active and the other comprising a water-in-oil emulsion of an aqueous solution of the polymeric co-gellant, the two emulsions being mixed to give the final product as a 'dual emulsion'.

### Examples

In the following examples, comparative examples are indicated by letters and examples according to the invention are indicated by numbers. All amounts are percentages by weight of the total composition.

**Table 1: Compositions and sensory results**

| **Example:** | **A** | **1** | **B** | **2** |
|---|---|---|---|---|
| DC 245 (1) | 13.86 | 13.16 | 13.86 | 13.51 |
| CAP 40 (2) | 50 | 50 | 50 | 50 |
| Finsolv TN (3) | 2 | 2 | 2 | 2 |
| Gantrez S95 (4) | - | - | 2 | 2 |
| Aloxicol L (5) | 20 | 20 | 20 | 20 |
| Abil EM90 (6) | 0.2 | 0.2 | 0.2 | 0.2 |
| HDK H30 (7) | - | 0.7 | - | 0.35 |
| Fragrance | 0.6 | 0.6 | 0.6 | 0.6 |
| Water | To 100 | To 100 | To 100 | To 100 |
| **Sensory Results** | | | | |
| Coolness on application | 39 | 30 | 44 | 40 |
| Wetness on application | 22 | 19 | 24 | 22 |
| Wetness immediately following application | 30 | 20 | 33 | 27 |
| Wetness 2 minutes after application | 16 | 6 | 21 | 14 |

| | | | | |
|---|---|---|---|---|
| 1. Cyclomethicone, ex Dow Corning. 2. Propellant (butane, isobutane, propane), *ex* Color/BP. 3. Emollient (C₁₂₋₁₅ alkyl benzoate), ex Finetex. 4. Co-polymer of methylvinylether and maleic acid, ex ISP. 5. 50% w/w aqueous ACH solution (hence 10% ACH solids in the compositions), ex Guilini. 6. Emulsifier, cetyl dimethicone copolyol, ex Goldschmidt. 7. Particulate silica with surfaces hydrophobically-modified by silylation (degree of silylation: ca. 50%; carbon content: 1.8% by weight), ex Wacker. | | | | |

Examples A and 1 were prepared in the following manner. The DC245, Finsolv TN, and Abil EM90 were stirred together. The Aloxicol L and additional water were mixed and slowly added to the oils and emulsfier, the stirring rate being increased during the addition. Following further stirring/shearing, the perfume was added, with continued stirring/shearing at a high rate. In the preparation of Example 1, the silica was mixed into the composition both manually using a spatula/pallet knife and mechanically, by stirring/shearing at a high rate using appropriate equipment. The resulting bases were transferred to aluminium aerosol cans and gassed with the CAP 40 using standard procedures.

Examples B and 2 were each prepared as two separate emulsions, which were then mixed in the aerosol can and gassed. A first emulsion was prepared as described above for Examples A and 1. In a separate vessel, a second emulsion was formed in the following manner. The DC245, Finsolv TN, Abil EM90 were stirred together. The Gantrez S95 was slowly added to the oils and emulsfier as an aqueous solution, the stirring rate being increased during the addition. Following further stirring/shearing, the perfume was added, with continued stirring/shearing at a high rate. In the preparation of Example 2, silica was mixed into the composition both manually using a spatula/pallet knife and mechanically, by stirring/shearing at a high rate using appropriate equipment. The required amounts of the ACH emulsion and the Gantrez S-95 emulsion:were transferred to aluminium aerosol cans and gassed with the CAP 40 using standard procedures.

The tabulated sensory results were obtained using an expert sensory panel comprised of 15-20 female volunteers. The panellist self-applied the compositions for 2 seconds each and then made judgements upon the indicated parameters on a 0 to 100 line scale.

Further qualitative data were obtained regarding the topical application of the above example compositions. Compositions A and B felt very wet and appeared wet and shiny on the skin surface, whilst compositions 1 and 2 felt cool and dry and appeared dry and matt on the skin surface.

Further examples were prepared by the same procedure as used for Example 1, but using different silicas. The silicas used and qualitative data obtained following topical application of these further examples are shown in Table 2. Details of Example 1 are shown again for ease of reference.

**Table 2: Further compositions and observations on application**

| **Example:** | **C** | **3** | **1** |
|---|---|---|---|
| Silica used (1) | Aerosil 200 (2) | HDK H30RM (3) | HDK H30 (4) |
| Appearance on skin | dry and very white | slightly shiny | dry and matt |
| Feel on skin | cool and quite wet | cool and slightly wet | cool and dry |

| | | | |
|---|---|---|---|
| 1. The only compositional variable; each silica used at 0.7%. 2. Particulate silica (not hydrophobically-modified), ex Degussa. 3. Particulate silica with surfaces hydrophobically-modified by silylation (degree of silylation: >75%; carbon content: >2.5% by weight), ex Wacker. 4. As detailed below table 1. | | | |

Further examples were prepared using emulsifier at 0.3%. Details of these compositions and qualitative data obtained following their topical application are shown in Table 3. The compositions were prepared in a manner analogous to that used for Example 1 and the qualitative data were obtained in the same manner as those indicated in Table 2.

The results in Table 3 illustrate the superior sensory properties of a composition according to the invention when compared with an analogous example comprising a hydrophobically treated hectorite clay.

**Table 3: Further compositions and observations on application**

| **Example:** | **D** | **4** |
|---|---|---|
| DC 245 (1) | 13.16 | 13.16 |
| CAP 40 (1) | 50 | 50 |
| Finsolv TN (1) | 2 | 2 |
| Aloxicol L (1) | 20 | 20 |
| Abil EM90 (1) | 0.3 | 0.3 |
| HDK H30 (1) | - | 0.6 |
| Bentone 38V (2) | 0.6 | - |
| Fragrance | 0.6 | 0.6 |
| Water | To 100 | To 100 |
| **Observations on application** | | |
| Appearance on skin | Wet, shiny, and creamy white | Dry and matt |
| Feel on skin | Cool and quite wet | Cool and dry |

| | | |
|---|---|---|
| 1. As detailed below Table 1. 2. Hydrophically treated hectorite clay, *ex* NL Industries, Inc. | | |

Example 4 from Table 3 was also compared with a control composition lacking hydrophobically-modified silica or clay (Comparative Example E). Details of this experiment are given in Table 4, below.

Comparative Example E was prepared in a manner analogous to that used for Example 1 and the qualitative data were obtained in the same manner as those indicated in Table 2. The quantitative sensory results were obtained using an expert sensory panel of approximately 30 volunteers. This was a different panel to that used to generate the data in Table 1; however, the test protocol was similar.

**Table 4: Further compositions, observations, and qualitative sensory data**

| **Example:** | **E** | **4** |
|---|---|---|
| DC 245 (1) | 13.86 | 13.16 |
| CAP 40 (1) | 50 | 50 |
| Finsolv TN (1) | 2 | 2 |
| Aloxicol L (1) | 20 | 20 |
| Abil EM90 (1) | 0.2 (2) | 0.3 |
| HDK H30(1) | - | 0.6 |
| Fragrance | 0.6 | 0.6 |
| Water | To 100 | To 100 |
| **Observations on application** | | |
| Appearance on skin | Wet and shiny | Dry and matt |
| Feel on skin | Very wet | Cool and dry |
| **Quantitative sensory data** | | |
| Wetness during application | 53 | 27 |
| Wetness after application | 53 | 26 |

| | | |
|---|---|---|
| 1. As detailed below Table 1. 2. Less surfactant required in the absence of the hydrophobically-modified silica. | | |

The Examples of Table 4 were also compared with regard to antiperspirancy performance. In a standard 'hot room' test, the antiperspirancy performance of Example 4 was found to be 11.9% greater than the antiperspirancy performance of Comparative Example E. In a repeat of this test, Example 4 was found to be 17.4% superior.

Table 5 compares the composition and performance of Comparative Example E with that of two other Examples according to the invention. Examples 5 and 6 were prepared in a manner analogous to that used for Example 1 and the qualitative data were obtained in the same manner as those indicated in Table 2. The quantitative sensory results given were obtained using the same expert sensory panel and protocol as used to generate the data in Table 1; however, it is not appropriate to directly compare the data of Table 1 with those of Table 5.

The antiperspirancy performance of Examples 5 and 6 were also compared with that of Comparative Example E using the same standard 'hot room' test used to compare Example 4 with Comparative Example E. In independent tests, Example 5 was found to be 20% more effective than Comparative Example E and Example 6 was found to be 15% more effective than Comparative Example E.

**Table 5: Further compositions, observations, and qualitative sensory data**

| **Example:** | **E** | **5** | **6** |
|---|---|---|---|
| DC 245 (1) | 13.86 | 13.46 | 13.06 |
| CAP 40 (1) | 50 | 50 | 50 |
| Finsolv TN (1) | 2 | 2 | 2 |
| Aloxicol L (1) | 20 | 20 | 20 |
| Abil EM90 (1) | 0.2 (1) | 0.25 | 0.3 |
| HDK H30(1) | - | 0.35 | 0.7 |
| Fragrance | 0.6 | 0.6 | 0.6 |
| Water | To 100 | To 100 | To 100 |
| **Observations on application** | | | |
| Appearance on skin | Wet and shiny | Dry and matt | Dry and matt |
| Feel on skin | Very wet | Cool and dry | Cool and dry |
| **Quantitative sensory data:** Wetness... | | | Assessment not performed |
| on application | 23 | 20 | -- |
| immediately after appln. | 21 | 16 | -- |
| 1 min. after application | 19 | 14 | -- |
| 2 min. after application | 16 | 12 | -- |

| | | | |
|---|---|---|---|
| 1. As detailed below Table 4. | | | |

The additional Examples according to the invention detailed in Table 6 were prepared in a manner analogous to that used for Example 1. These Examples were also found to appear and feel dry when applied to the skin.

**Table 6: Further compositions**

| **Example:** | **7** | **8** | **9** |
|---|---|---|---|
| DC 245 (1) | 8.41 | 13.46 | 11.21 |
| CAP 40 (1) | 65 | 50 | 50 |
| Finsolv TN (1) | 2 | 2 | 2 |
| Aloxicol L (1) | 20 | 20 | 20 |
| Abil EM90 (1) | 0.3 | -- | 0.3 |
| DC 5200 (2) | -- | 0.25 | -- |
| DC 3225 C (3) | -- | -- | 2.5 |
| HDK H30(1) | 0.35 | 0.35 | 0.35 |
| Fragrance | 0.6 | 0.6 | 0.6 |
| Water | To 100 | To 100 | To 100 |

| | | | |
|---|---|---|---|
| (1) As detailed below Table 1. (2) Lauryl dimethicone copolyol, ex Dow Corning. (3) Cyclomethicone and PEG/PPG-18/18 dimethicone (10.5% non-volatile content; cyclotetrasiloxane (D4) content ≤67.5%). | | | |

Example 9 may also be prepared using DC 5225 C as the hydrophobically-modified silica. DC 5225 C is cyclopentasiloxane and 10.5% PEG/PPG-18/18 dimethicone (10.5% non-volatile content).

## Claims

1. An antiperspirant spray composition comprising an aqueous solution of antiperspirant active emulsified in an oily continuous phase with an emulsifier that is a non-ionic surfactant, **characterised in** comprising a hydrophobically-modified particulate silica which is silylated.

2. A composition according to claim 1, wherein the antiperspirant active is aluminium chlorohydrate.

3. A composition according to claim 1 or claim 2, wherein the antiperspirant active is present in an amount from 5 to 30% by weight of the composition.

4. A composition according to any of the preceding claims, wherein the aqueous solution of antiperspirant active is of concentration from 10 to 70% by weight.

5. A composition according to any of the preceding claims, wherein the proportion of dispersed phase is from 50 to 90% by weight of the composition excluding any volatile propellant that may be present.

6. A composition according to any of the preceding claims, wherein at least 90% of the droplets of the aqueous phase are from 1 to 25 microns in diameter.

7. A composition according to any of the preceding claims, wherein the oily continuous phase comprises a silicone oil.

8. A composition according to any of the preceding claims, wherein the oily continuous phase comprises from 10 to 50% by weight of the composition excluding any volatile propellant that may be present.

9. A composition according to any of the preceding claims, wherein the hydrophobically-modified particulate silica is suspended in the oily continuous phase.

10. A composition according to any of the preceding claims, wherein the degree of silylation is from 25 to 75%.

11. A composition according to any of the preceding claims, wherein the hydrophobically-modified particulate silica has terminal polydimethylsiloxane groups and/or trimethylsilane groups.

12. A composition according to any of the preceding claims, wherein the hydrophobically-modified particulate silica is based on fumed silica.

13. A composition according to any of the preceding claims, wherein the hydrophobically-modified particulate silica is present at from 0.1 to 3% by weight of the composition.

14. A composition according to claim 13, wherein the hydrophobically-modified particulate silica is present at from 0.2 to 0.7% by weight of the composition.

15. A composition according to any of the preceding claims, wherein the hydrophobically-modified particulate silica has a carbon content of from 0.5 to 2.5% by weight.

16. A composition according to any of the preceding claims, comprising an emulsifier or mixture of emulsifiers with an overall HLB value of from 2 to 10.

17. A composition according to any of the preceding claims, comprising an emulsifier that is a silicone derivative.

18. A composition according to claim 17, comprising a dimethicone copolyol silicone surfactant.

19. A composition according to claim 17 or 18, wherein the emulsifier that is a silicone derivative is present at from 0.1 to 5% by weight of the composition.

20. A composition according to claim 19, wherein the emulsifier that is a silicone derivative is present at from 0.25 to 0.4% by weight of the composition.

21. A composition according to any of the preceding claims, comprising an emollient oil.

22. A composition according to any of the preceding claims, comprising a polymeric co-gellant.

23. A composition according to any of the preceding claims, comprising a volatile propellant.

24. A composition according to any of the preceding claims, packaged in a plastic container.

25. A cosmetic method of delivering an antiperspirancy benefit comprising the spray application to the human body of a composition according to any of claims 1 to 24.

26. A method of manufacture of an antiperspirant spray composition comprising the emulsification of an aqueous solution of antiperspirant active in an oily continuous phase using an emulsifier that is a non-ionic surfactant, a hydrophobically-modified particulate silica which is silylated being added to the emulsion once formed accompanied by vigorous mixing.

## Patentansprüche

1. Antiperspirans-Spray-Zusammensetzung, die eine wässrige Lösung von Antiperspirans-Wirkstoff emulgiert in einer öligen kontinuierlichen Phase mit einem Emulgator, der ein nicht-ionisches oberflächenaktives Mittel ist, umfasst, **dadurch gekennzeichnet, dass** sie ein hydrophob modifiziertes partikuläres Siliciumdioxid, das silyliert ist, umfasst.

2. Zusammensetzung gemäß Anspruch 1, wobei der Antiperspirans-Wirkststoff Aluminiumchlorohydrat ist.

3. Zusammensetzung gemäß Anspruch 1 oder Anspruch 2, wobei der Antiperspirans-Wirkstoff in einer Menge von 5 bis 30 Gewichts-% der Zusammensetzung vorliegt.

4. Zusammensetzung gemäß einem der vorangehenden Ansprüche, wobei die wässrige Lösung von Antiperspirans-Wirkstoff eine Konzentration von 10 bis 70 Gewichts-% hat.

5. Zusammensetzung gemäß einem der vorangehenden Ansprüche, wobei der Anteil der dispergierten Phase 50 bis 90 Gewichts-% der Zusammensetzung ohne ein flüchtiges Treibmittel, das vorliegen kann, ist.

6. Zusammensetzung gemäß einem der vorangehenden Ansprüche, wobei wenigstens 90 % der Tröpfchen der wässrigen Phase einen Durchmesser von 1 bis 25 Mikrometer haben.

7. Zusammensetzung gemäß einem der vorangehenden Ansprüche, wobei die kontinuierliche Ölphase ein Silikonöl umfasst.

8. Zusammensetzung gemäß einem der vorangehenden Ansprüche, wobei die kontinuierliche Ölphase 10 bis 50 Gewichts-% der Zusammensetzung ohne ein flüchtiges Treibmittel, das vorliegen kann, ausmacht.

9. Zusammensetzung gemäß einem der vorangehenden Ansprüche, wobei das hydrophob modifizierte partikuläre Siliciumdioxid in der kontinuierlichen Ölphase suspendiert ist.

10. Zusammensetzung gemäß einem der vorangehenden Ansprüche, wobei der Silylierungsgrad 25 bis 75 % ist.

11. Zusammensetzung gemäß einem der vorangehenden Ansprüche, wobei das hydrophob modifizierte partikuläre Siliciumdioxid terminale Polydimethylsiloxan-Gruppen und/oder Trimethylsilan-Gruppen hat.

12. Zusammensetzung gemäß einem der vorangehenden Ansprüche, wobei das hydrophob modifizierte partikuläre Siliciumdioxid auf Basis von hoch dispersem Siliciumdioxid ist.

13. Zusammensetzung gemäß einem der vorangehenden Ansprüche, wobei das hydrophob modifizierte partikuläre Siliciumdioxid mit 0,1 bis 3 Gewichts-% der Zusammensetzung vorliegt.

14. Zusammensetzung gemäß Anspruch 13, wobei das hydrophob modifizierte partikuläre Siliciumdioxid mit 0,2 bis 0,7 Gewichts-% der Zusammensetzung vorliegt.

15. Zusammensetzung gemäß einem der vorangehenden Ansprüche, wobei das hydrophob modifizierte partikuläre Siliciumdioxid einen Kohlenstoffgehalt von 0,5 bis 2,5 Gewichts-% hat.

16. Zusammensetzung gemäß einem der vorangehenden Ansprüche, die einen Emulgator oder ein Gemisch von Emulgatoren mit einem Gesamt-HLB-Wert von 2 bis 10 umfasst.

17. Zusammensetzung gemäß einem der vorangehenden Ansprüche, die einen Emulgator umfasst, der ein Silikonderivat ist.

18. Zusammensetzung gemäß Anspruch 17, die ein Dimethicon-copolyolsilikon als oberflächenaktives Mittel umfasst.

19. Zusammensetzung gemäß Anspruch 17 oder 18, wobei der Emulgator, der ein Silikonderivat ist, mit 0,1 bis 5 Gewichts-% der Zusammensetzung vorliegt.

20. Zusammensetzung gemäß Anspruch 19, wobei der Emulgator, der ein Silikonderivat ist, mit 0,25 bis 0,4 Gewichts-% der Zusammensetzung vorliegt.

21. Zusammensetzung gemäß einem der vorangehenden Ansprüche, die ein weichmachendes Öl umfasst.

22. Zusammensetzung gemäß einem der vorangehenden Ansprüche, die ein polymeres Co-Geliermittel umfasst.

23. Zusammensetzung gemäß einem der vorangehenden Ansprüche, die ein flüchtiges Treibmittel umfasst.

24. Zusammensetzung gemäß einem der vorangehenden Ansprüche, die in einem Kunststoffbehälter verpackt ist.

25. Kosmetisches Verfahren zur Abgabe eines Antiperspirans-Wirkstoffs, umfassend die Spray-Anwendung einer Zusammensetzung gemäß einem der Ansprüche 1 bis 24 auf den menschlichen Körper.

26. Verfahren zur Herstellung einer Antiprspirans-Spray-Zusammensetzung, umfassend die Emulgierung einer wässrigen Lösung von Antiperspirans-Wirkstoff in einer kontinuierlichen Ölphase unter Verwendung eines Emulgators, der ein nicht-ionisches oberflächenaktives Mittel ist, wobei ein hydrophob modifiziertes partikuläres Siliciumdioxid, das silyliert ist, der Emulsion, sobald sie gebildet ist, begleitet von kräftigem Mischen, zugesetzt wird.

## Revendications

1. Composition pour spray anti-transpirant comprenant une solution aqueuse d'anti-transpirant actif émulsifiée dans une phase continue huileuse avec un émulsifiant qui est un agent tensioactif non ionique, **caractérisée en ce qu'**elle comprend une silice particulaire modifiée hydrophobiquement qui est silylée.

2. Composition selon la revendication 1, dans laquelle l'anti-transpirant actif est un chlorhydrate d'aluminium.

3. Composition selon la revendication 1 ou la revendication 2, dans laquelle l'anti-transpirant actif est présent en une quantité de 5 à 30 % en poids de la composition.

4. Composition selon l'une quelconque des revendications précédentes, dans laquelle la solution aqueuse d'anti-transpirant actif est à une concentration de 10 à 70 % en poids.

5. Composition selon l'une quelconque des revendications précédentes, dans laquelle la proportion de la phase dispersée est de 50 à 90 % en poids de la composition, exclusion faite de tout gaz propulseur volatil susceptible d'être présent.

6. Composition selon l'une quelconque des revendications précédentes, dans laquelle au moins 90 % des gouttelettes de la phase aqueuse ont un diamètre de 1 à 25 microns.

7. Composition selon l'une quelconque des revendications précédentes, dans laquelle la phase continue huileuse comprend une huile silicone.

8. Composition selon l'une quelconque des revendications précédentes, dans laquelle la phase continue huileuse représente de 10 à 50 % en poids de la composition, exclusion faite de tout gaz propulseur volatil susceptible d'être présent.

9. Composition selon l'une quelconque des revendications précédentes, dans laquelle la silice particulaire modifiée hydrophobiquement est en suspension dans la phase continue huileuse.

10. Composition selon l'une quelconque des revendications précédentes, dans laquelle le degré de silylation est de 25 à 75 %.

11. Composition selon l'une quelconque des revendications précédentes, dans laquelle la silice particulaire modifiée hydrophobiquement possède des groupes polydiméthylsiloxane et/ou des groupes triméthylsilane terminaux.

12. Composition selon l'une quelconque des revendications précédentes, dans laquelle la silice particulaire modifiée hydrophobiquement est à base de fumée de silice.

13. Composition selon l'une quelconque des revendications précédentes, dans laquelle la silice particulaire modifiée hydrophobiquement est présente à raison de 0,1 à 3 % en poids de la composition.

14. Composition selon la revendication 13, dans laquelle la silice particulaire modifiée hydro-phobiquement est présente à raison de 0,2 à 0,7 % en poids de la composition.

15. Composition selon l'une quelconque des revendications précédentes, dans laquelle la silice particulaire modifiée hydrophobiquement a une teneur en carbone de 0,5 à 2,5 % en poids.

16. Composition selon l'une quelconque des revendications précédentes, comprenant un émulsifiant ou un mélange d'émulsifiants ayant un indice HLB global de 2 à 10.

17. Composition selon l'une quelconque des revendications précédentes, comprenant un émulsifiant qui est un dérivé de silicone.

18. Composition selon la revendication 17 comprenant un tensioactif de silicone diméthicone copolyol.

19. Composition selon les revendications 17 ou 18, dans laquelle l'émulsifiant qui est un dérivé de silicone est présent à raison de 0,1 à 5 % en poids de la composition.

20. Composition selon la revendication 19, dans laquelle l'émulsifiant qui est un dérivé de silicone est présent à raison de 0,25 à 0,4 % en poids de la composition.

21. Composition selon l'une quelconque des revendications précédentes, comprenant une huile émolliente.

22. Composition selon l'une quelconque des revendications précédentes, comprenant un cogélifiant de nature polymère.

23. Composition selon l'une quelconque des revendications précédentes, comprenant un gaz propulseur volatil.

24. Composition selon l'une quelconque des revendications précédentes, conditionnée dans un récipient en plastique.

25. Procédé cosmétique de délivrance d'un bénéfice anti-transpirant comprenant l'application par spray au corps humain d'une composition selon l'une quelconque des revendications 1 à 24.

26. Procédé de fabrication d'une composition pour spray anti-transpirant comprenant l'émulsification d'une solution aqueuse d'anti-transpirant actif dans une phase continue huileuse au moyen d'un émulsifiant qui est un agent tensioactif non ionique, et l'ajout d'une silice particulaire modifiée hydrophobiquement qui est silylée, étant additionnel à l'émulsion, une fois formée, accompagné d'un mélange vigoureux.
